# EUROPEAN PATENT APPLICATION

(11) **EP 3 517 608 A1**
(43) Date of publication of application: **31.07.2019**
(21) Application number: 18305083.0
(22) Date of filing: 30.01.2018
(51) Int. Cl.: C12N 9/14, C08J 11/10, C12N 9/18

(54) **NEW POLYPEPTIDES HAVING A POLYESTER DEGRADING ACTIVITY AND USES THEREOF**

(71) Applicant: Carbios, 63360 Saint-Beauzire (FR)
(72) Inventor: ZIMMERMANN, Wolfgang, 04109 Leipzig (DE); WEI, Ren, 04451 Borsdorf (DE); HILLE, Patrick, 04277 Leipzig (DE); OESER, Thorsten, 04229 Leipzig (DE); SCHMIDT, Juliane, 04177 Leipzig (DE)
(74) Representative: Cabinet Becker et Associés

(57) **Abstract**

The present invention relates to novel polyester hydrolases, comprising an amino acid sequence having at least 75%, 80%, 85%, 90%, 95%, 99% or 100% identity to the full length amino acid sequence set forth in SEQ ID N°1, and having a polyester degrading activity, particularly a polyethylene terephthalate degrading activity.

SEQUENCE LISTING

<110>  CARBIOS

<120>  NEW POLYPEPTIDES HAVING A POLYESTER DEGRADING ACTIVITY AND USES
       THEREOF

<130>  B2672EP00

<160>  4

<170>  PatentIn version 3.5

<210>  1
<211>  259
<212>  PRT
<213>  artificial sequence

<220>
<223>  polyester degrading enzyme

<400>  1

Met Ala Asn Pro Tyr Glu Arg Gly Pro Asp Pro Thr Glu Ser Ser Ile
1               5                   10                  15

Glu Ala Val Arg Gly Pro Phe Ala Val Ala Gln Thr Thr Val Ser Arg
            20                  25                  30

Leu Gln Ala Asp Gly Phe Gly Gly Gly Thr Ile Tyr Tyr Pro Thr Asp
        35                  40                  45

Thr Ser Gln Gly Thr Phe Gly Ala Val Ala Ile Ser Pro Gly Phe Thr
    50                  55                  60

Ala Gly Gln Glu Ser Ile Ala Trp Leu Gly Pro Arg Ile Ala Ser Gln
65                  70                  75                  80

Gly Phe Val Val Ile Thr Ile Asp Thr Ile Thr Arg Leu Asp Gln Pro
                85                  90                  95

Asp Ser Arg Gly Arg Gln Leu Gln Ala Ala Leu Asp His Leu Arg Thr
            100                 105                 110

Asn Ser Val Val Arg Asn Arg Ile Asp Pro Asn Arg Met Ala Val Met
        115                 120                 125

Gly His Ser Met Gly Gly Gly Gly Ala Leu Ser Ala Ala Ala Asn Asn
    130                 135                 140

Thr Ser Leu Glu Ala Ala Ile Pro Leu Gln Gly Trp His Thr Arg Lys
145                 150                 155                 160

Asn Trp Ser Ser Val Arg Thr Pro Thr Leu Val Val Gly Ala Gln Leu
                165                 170                 175

Asp Thr Ile Ala Pro Val Ser Ser His Ser Glu Ala Phe Tyr Asn Ser
            180                 185                 190

Leu Pro Ser Asp Leu Asp Lys Ala Tyr Met Glu Leu Arg Gly Ala Ser
        195                 200                 205

His Leu Val Ser Asn Thr Pro Asp Thr Thr Thr Ala Lys Tyr Ser Ile
    210                 215                 220

Ala Trp Leu Lys Arg Phe Val Asp Asp Asp Leu Arg Tyr Glu Gln Phe
225                 230                 235                 240

Leu Cys Pro Ala Pro Asp Asp Phe Ala Ile Ser Glu Tyr Arg Ser Thr
                245                 250                 255

Cys Pro Phe

<210>  2
<211>  259
<212>  PRT
<213>  artificial sequence

<220>
<223>  polyester degrading enzyme

<400>  2

Met Glu Asn Pro Tyr Glu Arg Gly Pro Asp Pro Thr Glu Ser Ser Ile
1               5                   10                  15

Glu Ala Val Arg Gly Pro Phe Ala Val Ala Gln Thr Thr Val Ser Arg
            20                  25                  30

Leu Gln Ala Asp Gly Phe Gly Gly Gly Thr Ile Tyr Tyr Pro Thr Asp
        35                  40                  45

Thr Ser Gln Gly Thr Phe Gly Ala Val Ala Ile Ser Pro Gly Phe Thr
    50                  55                  60

Ala Gly Gln Glu Ser Ile Ala Trp Leu Gly Pro Arg Ile Ala Ser Gln
65                  70                  75                  80

Gly Phe Val Val Ile Thr Ile Asp Thr Ile Thr Arg Leu Asp Gln Pro
                85                  90                  95

Asp Ser Arg Gly Arg Gln Leu Gln Ala Ala Leu Asp His Leu Arg Thr

100                 105                 110

Asn Ser Val Val Arg Asn Arg Ile Asp Pro Asn Arg Met Ala Val Met
        115                 120                 125

Gly His Ser Met Gly Gly Gly Gly Ala Leu Ser Ala Ala Ala Asn Asn
    130                 135                 140

Thr Ser Leu Glu Ala Ala Ile Pro Leu Gln Gly Trp His Thr Arg Lys
145                 150                 155                 160

Asn Trp Ser Ser Val Arg Thr Pro Thr Leu Val Val Gly Ala Gln Leu
                165                 170                 175

Asp Thr Ile Ala Pro Val Ser Ser His Ser Glu Ala Phe Tyr Asn Ser
            180                 185                 190

Leu Pro Ser Asp Leu Asp Lys Ala Tyr Met Glu Leu Arg Gly Ala Ser
        195                 200                 205

His Phe Val Ser Asn Thr Pro Asp Thr Thr Thr Ala Lys Tyr Ser Ile
    210                 215                 220

Ala Trp Leu Lys Arg Phe Val Asp Asn Asp Leu Arg Tyr Glu Gln Phe
225                 230                 235                 240

Leu Cys Pro Ala Pro Asp Asp Phe Ala Ile Ser Glu Tyr Arg Ala Thr
                245                 250                 255

Cys Pro Phe

<210>  3
<211>  777
<212>  DNA
<213>  artificial sequence

<220>
<223>  nucleic acid encoding polypeptide

<400>  3
atggcgaacc cgtacgagcg cgggcccgat cccaccgagt cgagcatcga ggccgtccgc      60

gggccgttcg ccgtggccca gacgacggtg tcgaggctcc aggccgacgg cttcggcggc     120

gggaccatct actacccgac cgacacgagc cagggcacct tcggtgcggt ggcgatctcg     180

ccggggttca cggcgggcca ggagagcatc gcctggctcg gcccccgcat cgcgtcgcag     240

ggcttcgtgg tgatcacgat cgacacgatc acgcgcctcg accagcccga cagccggggt     300

cgccagctgc aggccgcgct cgaccacctg cgcaccaaca gcgtcgtgcg caaccggatc     360

gacccgaacc ggatggcggt catgggccac tcgatgggcg gcggcggggc gctgtccgcc 420

gcggcgaaca acacgagcct cgaggccgcc atcccgctgc agggctggca cacccggaag 480

aactggtcga gcgtgcggac gccgaccctg gtggtcgggg cccagctcga caccatcgcg 540

ccggtgagct cgcactcgga ggccttctac aacagcctgc cgagcgacct cgacaaggcg 600

tacatggagc tccgcggggc cagccacctc gtgtcgaaca cgcccgacac gacgaccgcg 660

aagtacagca tcgcctggct caagcggttc gtcgacgacg acctccgcta cgagcagttc 720

ctgtgcccgg cgccggacga cttcgcgatc tccgagtacc gctccacctg cccgttc 777

<210> 4
<211> 777
<212> DNA
<213> artificial sequence

<220>
<223> nucleic acid encoding polypeptide

<400> 4
atggagaacc cgtacgagcg cgggcccgat cccaccgagt cgagcatcga ggccgtccgc 60

gggccgttcg ccgtggccca gacgacggtg tcgaggctcc aggccgacgg cttcggcggc 120

gggaccatct actacccgac cgacacgagc cagggcacct tcggtgcggt ggcgatctcg 180

ccggggttca cggcgggcca ggagagcatc gcctggctcg gcccccgcat cgcgtcgcag 240

ggcttcgtgg tgatcacgat cgacacgatc acgcgcctcg accagcccga cagccggggt 300

cgccagctgc aggccgcgct cgaccacctg cgcaccaaca gcgtcgtgcg caaccggatc 360

gacccgaacc ggatggcggt catgggccac tcgatgggcg gcggcggggc gctgtccgcc 420

gcggcgaaca acacgagcct cgaggccgcc atcccgctgc agggctggca cacccggaag 480

aactggtcga gcgtgcggac gccgaccctg gtggtcgggg cccagctcga caccatcgcg 540

ccggtgagct cgcactcgga ggccttctac aacagcctgc cgagcgacct cgacaaggcg 600

tacatggagc tccgcggggc cagccacttc gtgtcgaaca cgcccgacac gacgaccgcg 660

aagtacagca tcgcctggct caagcggttc gtcgacaacg acctccgcta cgagcagttc 720

ctctgcccgg cgccggacga cttcgccatc tccgagtacc gggccacctg cccgttc 777

## Description

The present invention relates to novel polypeptides having an enzymatic activity, particularly a polyester degrading activity, and the uses thereof. The invention also relates to methods of producing such polypeptides, coding nucleic acid molecule, recombinant cells and methods of degrading polyester-containing material with use of such polypeptides. The polypeptides of the invention are particularly suited to degrade polyethylene terephthalate, and material containing polyethylene terephthalate, as plastic material.

### BACKGROUND

Polyesters are used in a large number of technical fields, in particular in the form of plastic material, from food packaging to the medical field, via clothing, the automobile industry, etc. As an example, certain polyesters such as polyethylene terephthalate (PET) are used in the manufacture of clothes and packaging, but also in the form of a thermoset resin for the manufacture of automobile or other parts.

As a consequence, the production of polyester containing plastics has increased dramatically over the last decades. More than 50% of these plastics are used for single-use disposable applications, such as packaging, agricultural films, disposable consumer items or for short-lived products that are discarded within a year of manufacture. Regrettably, plastics may persist for decades depending on local environmental factors, like levels of ultraviolet light exposure, temperature, presence of suitable microorganisms, etc. As a consequence, substantial quantities of plastics are piling up in landfill sites and in natural habitats worldwide, generating increasing environmental problems.

One solution to reduce environmental and economic impacts correlated to the accumulation of plastic is recycling wherein plastic material is mechanically reprocessed to manufacture new products. However, the actual recycling processes use huge amounts of electricity, particularly during the extruding step, and the equipment used is also expensive, leading to high prices which may be non-competitive compared to virgin plastic.

Various solutions were proposed in order to improve the recycling of such plastic such as chemical recycling or enzymatic recycling, as described in WO2014/079844, WO2015/173265 or WO2017/198786. Enzymatic degradation is the preferred treatment to decrease plastic waste accumulation. Indeed, enzymes may be used both to accelerate hydrolysis of plastics and to allow the recovery of the chemical constituents (i.e., monomers and/or oligomers) of the polymer. The resulting monomers or oligomers may then be used to re-manufacture plastic or to make other synthetic chemicals. Thus, the depolymerization of polymers contained in a plastic product by enzymes is of great interest, as an alternative to the existing and unsatisfactory processes.

In this context, several PET hydrolases have been identified as candidate degrading enzymes, such as referenced in Wei et al. 2017 ("Microbial enzymes for the recycling of recalcitrant petroleum-based plastics: how far are we?". Microbial Biotechnology 2017 March 28; and "Biocatalysis as a green route for recycling the recalcitrant plastic polyethylene terephthalate" Microbial Biotechnology. 2017 April 12). Some of them have an effective degrading activity and may be used for biocatalytic depolymerization of PET. However, most often, these polyester hydrolases do neither have enough efficiency on high crystalline and bottle-grade PET materials nor enough stability to be employed in a competitive degrading process.

In view of the foregoing, there is a need for novel enzymes active in the degradation of polyesters, and more particularly of PET, including polyesters and PET contained in plastic products.

### SUMMARY OF THE INVENTION

Work conducted by the applicant has led to the identification of novel polypeptides having a polyester degrading activity, and more specifically a PET degrading activity. These polypeptides had never been reported or isolated in the art and bring substantial improvements to the development of industrial processes of degrading polyester containing material.

Then, the invention stems inter alia from the identification of these new polypeptides having the remarkable properties of degrading polyester and/or optimizing polyester degradation. Based on these unexpected properties, the polypeptides of the invention may be successfully used for obtaining, even on an industrial scale, the degradation of polyesters contained in a plastic product.

It is therefore an object of the present invention to provide isolated polypeptides comprising an amino acid sequence having at least 75%, 80%, 85%, 90%, 95%, 99% or 100% identity to the full length amino acid sequence set forth in SEQ ID N°1, and having a polyester degrading activity.

Particularly, the isolated polypeptides of the invention have at least 75%, 80%, 85%, 90%, 95%, or 99% identity to the full length amino acid sequence set forth in SEQ ID N°1 and comprise at least one substitution at a position selected from A2, L210, D233 or S255 or any combinations thereof, wherein the positions are numbered by reference to the amino acid sequence set forth in SEQ ID N°1. In a particular embodiment, the polypeptides comprise at least one substitution selected from A2E, L210F, D233N or S255A.

In a specific embodiment, the invention relates to a polypeptide having the amino acid sequence as set forth in SEQ ID N°1 or in SEQ ID N°2.

It is another object of the invention to provide a nucleic acid encoding an isolated polypeptide of the invention. The present invention also relates to an expression cassette or an expression vector comprising said nucleic acid, and to a host cell comprising said nucleic acid, expression cassette or vector.

It is a further object of the invention to provide a method of producing an isolated polypeptide of the invention comprising:
(a) culturing the host cell according to the invention under suitable conditions to express the nucleic acid encoding the polypeptide; and optionally
(b) recovering said polypeptide from the cell culture.

The present invention also relates to a method of degrading a plastic product containing at least one polyester, preferably polyethylene terephthalate (PET), comprising
(a) contacting the plastic product with the polypeptide or the host cell according to the invention, thereby degrading the plastic product; and optionally
(b) recovering monomers and/or oligomers.

The present invention also relates to a method of degrading polyester, preferably polyethylene terephthalate (PET) comprising
(a) contacting the polyester with the polypeptide or the host cell according to the invention, thereby degrading the polyester; and optionally
(b) recovering monomers and/or oligomers.

The present invention further relates to the use of a polypeptide as described above for the degradation of a plastic product containing at least one polyester, preferably at least PET.

### DETAILED DESCRIPTION OF THE INVENTION

The invention relates, generally, to isolated polypeptides which are able to depolymerize polyesters, such as polyethylene terephthalate (PET), polyester polyurethane and polycaprolactone (PCL), more preferably polyethylene terephthalate, and the uses thereof, particularly for degrading polyester-containing material, such as plastic products.

The polypeptides of the invention are active and particularly stable at a temperature range from 20°C to 90°C, preferably from 50°C to 75°C, more preferably from 65°C to 75°C. Particularly, these polypeptides show less than 70% of loss of activity, preferably less than 60%, more preferably less than 50%, more preferably less than 30%, even more preferably less than 25% of loss of activity when incubated during 24h at a temperature range from 65°C to 75°C, as compared to same polypeptide stored during 24h at a temperature of 4°C. The stability of such polypeptides, and thus the preservation of the activity at a temperature range from 65°C to 75°C is particularly advantageous in order to perform competitive process at such temperatures.

These polypeptides, or their coding nucleic acid sequences, may also be used to create recombinant microorganisms, which may serve to cause degradation of polyester-containing materials.

The following is a description of the present invention, including preferred embodiments thereof given in general terms. The present invention is further exemplified in the disclosure given under the heading "Examples" herein below, which provides experimental data supporting the invention and means of performing the invention.

### Definitions

The present disclosure will be best understood by reference to the following definitions.

The term *"isolated"* means that the material is removed from its original environment (e.g., the natural environment). For instance, an isolated polypeptide is typically devoid of at least some polypeptides or other constituents of the cells to which it is normally associated or with which it is normally admixed or in solution. An isolated polypeptide includes said naturally-produced polypeptide in a purified or partially purified form, the recombinant polypeptide, the polypeptide which is expressed or secreted by a host cell, as well as the polypeptide in a host cell or culture or extract thereof. In a preferred aspect, the polypeptide is at least 10% pure, preferably at least 50% pure, more preferably at least 60%, 70%, 80%, 90% pure, as determined by SDS-PAGE. A purity less than 100% denotes herein a polypeptide preparation that contains other polypeptide material that it is natively or recombinantly associated. In relation to a nucleic acid, the term isolated or purified indicates e.g., that the nucleic acid is not in its natural genomic context (e.g., in a vector, an expression cassette, linked to a promoter, or artificially introduced in a heterologous host cell).

Herein, the terms *"peptide", "polypeptide", "protein"* refer to a chain of amino acids linked by peptide bonds, regardless of the number of amino acids forming said chain. The amino acids are herein represented by their one-letter or three-letters code according to the following nomenclature: A: alanine (Ala); C: cysteine (Cys); D: aspartic acid (Asp); E: glutamic acid (Glu); F: phenylalanine (Phe); G: glycine (Gly); H: histidine (His); I: isoleucine (Ile); K: lysine (Lys); L: leucine (Leu); M: methionine (Met); N: asparagine (Asn); P: proline (Pro); Q: glutamine (Gln); R: arginine (Arg); S: serine (Ser); T: threonine (Thr); V: valine (Val); W: tryptophan (Trp ) and Y: tyrosine (Tyr).

As used herein, the term *"sequence identity"* or *"identity"* refers to the number (or fraction expressed as a percentage %) of matches (identical amino acid residues) between two polypeptide sequences. The sequence identity is determined by comparing the sequences when aligned so as to maximize overlap and identity while minimizing sequence gaps. In particular, sequence identity may be determined using any of a number of mathematical global or local alignment algorithms, depending on the length of the two sequences. Sequences of similar lengths are preferably aligned using a global alignment algorithms (e.g. Needleman and Wunsch algorithm; Needleman and Wunsch, 1970) which aligns the sequences optimally over the entire length, while sequences of substantially different lengths are preferably aligned using a local alignment algorithm (e.g. Smith and Waterman algorithm (Smith and Waterman, 1981) or Altschul algorithm (Altschul et al., 1997; Altschul et al., 2005)). Alignment for purposes of determining percent amino acid sequence identity can be achieved in various ways that are within the skill in the art, for instance, using publicly available computer software available on internet web sites such as http://blast.ncbi.nlm.nih.gov/ or http://www.ebi.ac.uk/Tools/emboss/). Those skilled in the art can determine appropriate parameters for measuring alignment, including any algorithms needed to achieve maximal alignment over the full length of the sequences being compared. For purposes herein, % amino acid sequence identity values refers to values generated using the pair wise sequence alignment program EMBOSS Needle that creates an optimal global alignment of two sequences using the Needleman-Wunsch algorithm, wherein all search parameters are set to default values, i.e. Scoring matrix = BLOSUM62, Gap open = 10, Gap extend = 0.5, End gap penalty = false, End gap open = 10 and End gap extend = 0.5.

The term *"modification"* or *"alteration"* as used herein in relation to a position or amino acid residue means that the amino acid in the particular position has been modified compared to the amino acid as set forth in SEQ ID N°1.

A *"substitution"* means that an amino acid residue is replaced by another amino acid residue. Preferably, the term "substitution" refers to the replacement of an amino acid residue by another residues selected from (i) the naturally-occurring standard 20 amino acid residues, (ii) rare naturally occurring amino acid residues (e.g. hydroxyproline, hydroxylysine, allohydroxylysine, 6-N-methylysine, N-ethylglycine, N-methylglycine, N-ethylasparagine, allo-isoleucine, N-methylisoleucine, N-methylvaline, pyroglutamine, aminobutyric acid, ornithine, norleucine, norvaline), and (iii) non-naturally occurring amino acid residues, often made synthetically, (e.g. cyclohexyl-alanine). Preferably, the term *"substitution"* refers to the replacement of an amino acid residue by another selected from the naturally-occurring standard 20 amino acid residues (G, P, A, V, L, I, M, C, F, Y, W, H, K, R, Q, N, E, D, S and T). The sign "+" indicates a combination of substitutions. In the present document, the following terminology is used to designate a substitution: L23A denotes that amino acid residue Leucine (L) at position 23 of the parent sequence is changed to an Alanine (A). A121V/I/M denotes that amino acid residue Alanine (A) at position 121 of the parent sequence is substituted by one of the following amino acids: Valine (V), Isoleucine (I), or Methionine (M). The substitution can be a conservative or non-conservative substitution. Examples of conservative substitutions are within the groups of basic amino acids (arginine, lysine and histidine), acidic amino acids (glutamic acid and aspartic acid), polar amino acids (glutamine, asparagine and threonine), hydrophobic amino acids (methionine, leucine, isoleucine, cysteine and valine), aromatic amino acids (phenylalanine, tryptophan and tyrosine), and small amino acids (glycine, alanine and serine).

Unless otherwise specified, the positions disclosed in the present application are numbered by reference to the amino acid sequence set forth in SEQ ID N°1.

The term *"recombinant"* refers to a nucleic acid construct, a vector, a polypeptide or a cell produced by genetic engineering.

The term *"expression",* as used herein, refers to any step involved in the production of a polypeptide including, but being not limited to, transcription, post-transcriptional modification, translation, post-translational modification, and secretion.

The term *"expression cassette"* denotes a nucleic acid construct comprising a coding region, i.e. a nucleic acid of the invention, and a regulatory region, i.e. comprising one or more control sequences, operably linked.

As used herein, the term *"expression vector"* means a DNA or RNA molecule that comprises an expression cassette of the invention. Preferably, the expression vector is a linear or circular double stranded DNA molecule.

A *"polymer"* refers to a chemical compound or mixture of compounds whose structure is constituted of multiple monomers (repeat units) linked by covalent chemical bonds. Within the context of the invention, the term polymer includes natural or synthetic polymers, constituted of a single type of repeat unit (i.e., homopolymers) or of a mixture of different repeat units (i.e., copolymers or heteropolymers). In the context of the invention, a polymer refers more particularly to synthetic polymers. According to the invention, *"oligomers"* refer to molecules containing from 2 to about 20 monomers.

In the context of the invention, a *"polyester containing material"* or *"polyester containing product"* refers to a product, such as plastic product, comprising at least one polyester in crystalline, semi-crystalline or totally amorphous forms. In a particular embodiment, the polyester containing material refers to any item made from at least one plastic material, such as plastic sheet, tube, rod, profile, shape, film, massive block etc., which contains at least one polyester, and possibly other substances or additives, such as plasticizers, mineral or organic fillers. In another particular embodiment, the polyester containing material refers to a plastic compound, or plastic formulation, in a molten or solid state, suitable for making a plastic product.

In the present description, *"polyesters"* encompasses but is not limited to polyethylene terephthalate (PET), polytrimethylene terephthalate (PTT), polybutylene terephthalate (PBT), polyethylene isosorbide terephthalate (PEIT), polylactic acid (PLA), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA), polyethylene naphthalate (PEN), polyester polyurethane, and blends/mixtures of these polymers.

### New polyester hydrolases

The present invention is directed to new polypeptides having the ability to degrade plastics having ester bonds in their molecular structure. More particularly, the present invention discloses newly identified and isolated polypeptides that exhibit a polyesterase activity, i.e. a polyester degrading activity. In the context of the invention, the expression "polyester hydrolase" is used to refer to polypeptides having a polyester degrading activity. Interestingly, the polypeptides of the invention are capable of hydrolyzing ester bonds both in natural and man-made polyesters, and particularly in polyethylene terephthalate.

In a first aspect, the present invention relates to isolated polypeptides comprising an amino acid sequence having at least 75%, 80%, 85%, 90%, 95%, 99% or 100% identity to the full length amino acid sequence set forth in SEQ ID N°1, and having a polyester degrading activity.

In a particular embodiment, the isolated polypeptides comprise at least one amino acid modification, as compared to the amino acid sequence SEQ ID N°1, in an amino acid residue impacting the thermostability and/or the polyester degrading activity of said polypeptide, as compared to the polypeptide of SEQ ID N°1. Advantageously, such modification(s) increases thermostability and/or polyester degrading activity of the polypeptide.

Within the context of the invention, the term "increased thermostability" means an increased ability of a polypeptide to resist to changes in its chemical and/or physical structure at high temperatures, and more particularly at temperature between 50°C and 90°C, as compared to a polypeptide having the amino acid sequence set forth in SEQ ID N°1. Such an increase is typically of about 1-fold, 2-fold, 3-fold, 4-fold, 5-fold, or more. The thermostability of the polypeptide may be evaluated by the one skilled in the art, according to methods known per se in the art. For instance, thermostability can be assessed by analysis of the polypeptide folding using circular dichroism. Alternatively, or in addition, thermostability can be assessed by measuring the residual polyester degrading activity of the polypeptide after incubation at different temperatures. The ability to perform multiple rounds of polyester's degrading assays at different temperatures can also be evaluated. A rapid and valuable test may consist on the evaluation, by halo diameter measurement, of the polypeptide ability to degrade a solid polyester compound dispersed in an agar plate after incubation at different temperatures.

Within the context of the invention, the term "increased activity" or "increased degrading activity" means an increased ability of a polypeptide to degrade a plastic product or material, and more particularly a polyester-containing plastic product or material, as compared to a polypeptide having the amino acid sequence set forth in SEQ ID N°1. Such an increase is typically of about 1-fold, 2-fold, 3-fold, 4-fold, 5-fold, or more. The activity of a polypeptide may be evaluated by the one skilled in the art, according to methods known per se in the art. For instance, the activity can be assessed by the measurement of the specific polyester degrading activity rate, the measurement of the rate to degrade a solid polyester compound dispersed in an agar plate, or the measurement of the specific polyester's degradation activity rate in reactor.

In a particular embodiment, the isolated polypeptides comprise at least one substitution at a position selected from A2, L210, D233 or S255, wherein the positions are numbered by reference to the amino acid sequence set forth in SEQ ID N°1.

According to the invention, the targeted amino acid(s) may be replaced by any one of the other amino acids selected from standard naturally-occurring amino acid residues, rare naturally occurring amino acid residues and non-naturally occurring amino acid residue. The targeted amino acid(s) may be replaced by any one of the 19 other naturally-occurring amino acids.

In a particular embodiment, the isolated polypeptides have at least 75%, 80%, 85%, 90%, 95%, or 99% identity to the full length amino acid sequence set forth in SEQ ID N°1 and comprise at least one substitution as compared to SEQ ID N°1, selected from A2E, L210F, D233N or S255A. In another embodiment, the isolated polypeptides comprise at least two or more substitutions as compared to SEQ ID N°1, selected from A2E, L210F, D233N and S255A.

A particular example of a polypeptide of the invention comprises at least the combination of substitutions consisting of A2E + L210F + D233N + S255A (SEQ N°2). Interestingly, a polypeptide having the amino acid sequence SEQ ID N°2 exhibits an increased stability as compared to a polypeptide having the amino acid sequence SEQ ID N°1, at a temperature range from 65°C to 75°C.

In a particular embodiment, the polypeptide has the amino acid sequence as set forth in SEQ ID N°1. In another embodiment, the polypeptide has the amino acid sequence as set forth in SEQ ID N°2.

As stated above, the polypeptides of the invention have a polyester degrading activity (i.e., polyester hydrolases). More preferably, the polypeptides have an esterase activity, even more preferably a cutinase activity.

Preferably, the polyester hydrolases of the invention have a PET degrading activity. Alternatively, or in addition, the polyester hydrolases of the invention have a PCL degrading activity. Alternatively, or in addition, the polyester hydrolases of the invention have a polyester polyurethane degrading activity.

Advantageously, the polyester hydrolases of the invention exhibit a measurable polyester degrading activity at least in a range of temperatures from 20°C to 90°C, preferably from 40°C to 80°C, more preferably from 50°C to 80°C, even more preferably from 60°C to 80°C, even more preferably at 70°C, +/- 5°C. In a particular embodiment, the polyester hydrolases of the invention exhibit a measurable polyester degrading activity at least at 65°C. In another particular embodiment, the polyester hydrolases of the invention exhibit a measurable polyester degrading activity at least at 75°C. In a particular embodiment, the polyester degrading activity is still measurable at a temperature between 60°C and 90°C.

In a particular embodiment, the polyester hydrolases of the invention exhibit a measurable polyester hydrolase activity at least in a range of pH from 5 to 11, preferably in a range of pH from 6 to 9, more preferably in a range of pH from 6.5 to 9, even more preferably in a range of pH from 7.5 to 9, and particularly at pH 8.

The polyester hydrolase of the invention advantageously has a productivity of at least 0.02 g.mg⁻¹.h⁻¹, 0.05 g.mg⁻¹.h⁻¹, 0.1 g.mg⁻¹.h⁻¹, 0.15 g.mg⁻¹.h⁻¹, 0.2 g.mg⁻¹.h⁻¹. 0.5 g.mg⁻¹.h⁻¹, 1 g.mg⁻¹.h⁻¹, 1.5 g.mg⁻¹.h⁻¹ or 2 g.mg⁻¹.h⁻¹. By "productivity" is meant the amount of product of degradation (i.e., monomers) formed per unit of polyester hydrolase and per unit time, at a pH comprised between 6.5 and 9 and at a temperature of 70°C +/- 5°C, particularly at a temperature of 65°C or 75°C.

### Nucleic acids, expression cassette, vector, host cell

It is a further object of the invention to provide a nucleic acid encoding a polyester hydrolase as defined above.

As used herein, the term *"nucleic acid", "nucleic sequence," "polynucleotide", "oligonucleotide"* and *"nucleotide sequence"* are used interchangeably and refer to a sequence of deoxyribonucleotides and/or ribonucleotides. The nucleic acids can be DNA (cDNA or gDNA), RNA, or a mixture of the two. It can be in single stranded form or in duplex form or a mixture of the two. It can be of recombinant, artificial and/or synthetic origin and it can comprise modified nucleotides, comprising for example a modified bond, a modified purine or pyrimidine base, or a modified sugar. The nucleic acids of the invention can be in isolated or purified form, and made, isolated and/or manipulated by techniques known per se in the art, e.g., cloning and expression of cDNA libraries, amplification, enzymatic synthesis or recombinant technology. The nucleic acids can also be synthesized in vitro by well-known chemical synthesis techniques, as described in, e.g., Belousov (1997) Nucleic Acids Res. 25:3440-3444.

The invention also encompasses nucleic acids which hybridize, under stringent conditions, to a nucleic acid encoding a polyester hydrolase as defined above. Preferably, such stringent conditions include incubations of hybridization filters at about 42° C for about 2.5 hours in 2 X SSC/0.1%SDS, followed by washing of the filters four times of 15 minutes in 1 X SSC/0.1% SDS at 65° C. Protocols used are described in such reference as Sambrook et al. (Molecular Cloning: a Laboratory Manual, Cold Spring Harbor Press, Cold Spring Harbor N.Y. (1988)) and Ausubel (Current Protocols in Molecular Biology (1989)).

The invention also encompasses nucleic acids encoding polyester hydrolase of the invention, wherein the sequence of said nucleic acids, or a portion of said sequence at least, has been engineered using optimized codon usage.

A specific embodiment of this invention resides in an isolated nucleic acid encoding a polypeptide as defined above, comprising the sequence set forth in SEQ ID N°3.

Another specific embodiment of this invention resides in an isolated nucleic acid encoding a polypeptide as defined above, comprising the sequence set forth in SEQ ID N°4.

Alternatively, the nucleic acid according to the invention may be deduced from the sequence of the polyester hydrolase according to the invention and codon usage may be adapted according to the host cell in which the nucleic acid shall be transcribed. These steps may be carried out according to methods well known to one skilled in the art and some of which are described in the reference manual Sambrook et al. (Sambrook et al., 2001).

Nucleic acids of the invention may further comprise additional nucleotide sequences, such as regulatory regions, i.e., promoters, enhancers, silencers, terminators, signal peptides and the like that can be used to cause or regulate expression of the polypeptide in a selected host cell or system.

The present invention further relates to an expression cassette comprising a nucleic acid according to the invention operably linked to one or more control sequences that direct the expression of said nucleic acid in a suitable host cell. Typically, the expression cassette comprises, or consists of, a nucleic acid according to the invention operably linked to a control sequence such as transcriptional promoter and/or transcription terminator. The control sequence may include a promoter that is recognized by a host cell or an *in vitro* expression system for expression of a nucleic acid encoding a polyester hydrolase of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell. The control sequence may also be a transcription terminator, which is recognized by a host cell to terminate transcription. The terminator is operably linked to the 3'-terminus of the nucleic acid encoding the polyester hydrolase. Any terminator that is functional in the host cell may be used in the present invention. Typically, the expression cassette comprises, or consists of, a nucleic acid according to the invention operably linked to a transcriptional promoter and a transcription terminator.

The invention also relates to a vector comprising a nucleic acid or an expression cassette as defined above.

The term *"vector"* refers to DNA molecule used as a vehicle to transfer recombinant genetic material into a host cell. The major types of vectors are plasmids, bacteriophages, viruses, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences encoding a polypeptide. Generally, the regulatory elements that are present in an expression vector include a transcriptional promoter, a ribosome binding site, a terminator, and optionally present operator. Preferably, an expression vector also contains an origin of replication for autonomous replication in a host cell, a selectable marker, a limited number of useful restriction enzyme sites, and a potential for high copy number. Examples of expression vectors are cloning vectors, modified cloning vectors, specifically designed plasmids and viruses. Expression vectors providing suitable levels of polypeptide expression in different hosts are well known in the art. The choice of the vector will typically depend on the compatibility of the vector with the host cell into which the vector is to be introduced.

It is another object of the invention to provide a host cell comprising a nucleic acid, an expression cassette or a vector as described above. The present invention thus relates to the use of a nucleic acid, expression cassette or vector according to the invention to transform, transfect or transduce a host cell. The choice of the vector will typically depend on the compatibility of the vector with the host cell into which it must be introduced.

According to the invention, the host cell may be transformed, transfected or transduced in a transient or stable manner. The expression cassette or vector of the invention is introduced into a host cell so that the cassette or vector is maintained as a chromosomal integrant or as a self-replicating extra-chromosomal vector. The term "host cell" also encompasses any progeny of a parent host cell that is not identical to the parent host cell due to mutations that occur during replication. The host cell may be any cell useful in the production of a variant of the present invention, e.g., a prokaryote or a eukaryote. The prokaryotic host cell may be any Gram-positive or Gram-negative bacterium. The host cell may also be an eukaryotic cell, such as a yeast, fungal, mammalian, insect or plant cell. In a particular embodiment, the host cell is selected from the group of *Escherichia coli, Bacillus, Streptomyces, Trichoderma, Aspergillus, Saccharomyces, Pichia* or *Yarrowia.*

The nucleic acid, expression cassette or expression vector according to the invention may be introduced into the host cell by any method known by the skilled person, such as electroporation, conjugation, transduction, competent cell transformation, protoplast transformation, protoplast fusion, biolistic "gene gun" transformation, PEG-mediated transformation, lipid-assisted transformation or transfection, chemically mediated transfection, lithium acetate-mediated transformation, liposome-mediated transformation.

Optionally, more than one copy of a nucleic acid, cassette or vector of the present invention may be inserted into a host cell to increase production of the polypeptide.

In a particular embodiment, the host cell is a recombinant microorganism. The invention indeed allows the engineering of microorganisms with improved capacity to degrade polyester containing material. For instance, the sequence of the invention may be used to complement a wild type strain of a fungus, yeast or bacterium already known as able to degrade polyester, in order to improve and/or increase the strain capacity.

### Production of the polyester hydrolases

It is another object of the invention to provide a method of producing the polyester hydrolase of the invention, comprising expressing a nucleic acid encoding the polyester hydrolase and optionally recovering the polyester hydrolase.

In particular, the present invention relates to *in vitro* methods of producing a polyester hydrolase of the present invention comprising (a) contacting a nucleic acid, cassette or vector of the invention with an *in vitro* expression system; and (b) recovering the polyester hydrolase produced. *In vitro* expression systems are well-known by the person skilled in the art and are commercially available.

Preferably, the method of production comprises
(a) culturing a host cell that comprises a nucleic acid encoding a polyester hydrolase of the invention under conditions suitable to express the nucleic acid; and optionally
(b) recovering said polyester hydrolase from the cell culture.

Advantageously, the host cell is a recombinant *Bacillus,* recombinant *E. coli,* recombinant *Aspergillus,* recombinant *Trichoderma,* recombinant *Streptomyces,* recombinant *Saccharomyces,* recombinant *Pichia* or recombinant *Yarrowia lipolytica.*

The host cells are cultivated in a nutrient medium suitable for production of polypeptides, using methods known in the art. For example, the cell may be cultivated by shake flask cultivation, or small-scale or large-scale fermentation (including continuous, batch, fed- batch, or solid state fermentations) in laboratory or industrial fermentors performed in a suitable medium and under conditions allowing the polypeptide to be expressed and/or isolated. The cultivation takes place in a suitable nutrient medium, from commercial suppliers or prepared according to published compositions (e.g., in catalogues of the American Type Culture Collection).

If the polyester hydrolase is excreted into the nutrient medium, the polyester hydrolase can be recovered directly from the culture supernatant. Conversely, the polyester hydrolase can be recovered from cell lysates or after permeabilisation. The polyester hydrolase may be recovered using any method known in the art. For example, the polyester hydrolase may be recovered from the nutrient medium by conventional procedures including, but not limited to, collection, centrifugation, filtration, extraction, spray-drying, evaporation, or precipitation. Optionally, the polyester hydrolase may be partially or totally purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction to obtain substantially pure polypeptides.

The polyester hydrolase may be used as such, in purified form, either alone or in combinations with additional enzymes, to catalyze enzymatic reactions involved in the degradation and/or recycling of a polyester containing material, such as plastic products containing polyester. The polyester hydrolase may be in soluble form, or on solid phase. In particular, it may be bound to cell membranes or lipid vesicles, or to synthetic supports such as glass, plastic, polymers, filter, membranes, e.g., in the form of beads, columns, plates and the like.

### Composition

It is a further object of the invention to provide a composition comprising a polyester hydrolase or a host cell of the invention. In the context of the invention, the term "composition" encompasses any kind of compositions comprising a polyester hydrolase of the invention. In a particular embodiment, the polyester hydrolase is in isolated or at least partially purified form.

The composition may be liquid or dry, for instance in the form of a powder. In some embodiments, the composition is a lyophilisate. For instance, the composition may comprise the polyester hydrolase and/or recombinant cells encoding the polyester hydrolase of the invention or extract thereof, and optionally excipients and/or reagents etc. Appropriate excipients encompass buffers commonly used in biochemistry, agents for adjusting pH, preservatives such as sodium benzoate, sodium sorbate or sodium ascorbate, conservatives, protective or stabilizing agents such as starch, dextrin, arabic gum, salts, sugars e.g. sorbitol, trehalose or lactose, glycerol, polyethyleneglycol, polyethene glycol, polypropylene glycol, propylene glycol, sequestering agent such as EDTA, reducing agents, amino acids, a carrier such as a solvent or an aqueous solution, and the like. The composition of the invention may be obtained by mixing the polyester hydrolase with one or several excipients.

The composition of the invention may comprise from 0.1% to 99.9%, preferably from 0.1% to 50%, more preferably from 0.1% to 30%, even more preferably from 0.1% to 5% by weight of the polyester hydrolase of the invention and from 0.1% to 99.9%, preferably from 50% to 99.9%, more preferably from 70% to 99.9%, even more preferably from 95% to 99.9% by weight of excipient(s). A preferred composition comprises between 0.1 and 5% by weight of the polyester hydrolase of the invention.

In a particular embodiment, the composition may further comprise additional polypeptide(s) exhibiting an enzymatic activity. The amounts of polyester hydrolase of the invention will be easily adapted by those skilled in the art depending e.g., on the nature of the polyester containing material to degrade and/or the additional enzymes/polypeptides contained in the composition.

In a particular embodiment, the polyester hydrolase of the invention is solubilized in an aqueous medium together with one or several excipients, especially excipients which are able to stabilize or protect the polypeptide from degradation. For instance, the polyester hydrolase of the invention may be solubilized in water, eventually with additional components, such as glycerol, sorbitol, dextrin, starch, glycol such as propanediol, salt, etc. The resulting mixture may then be dried so as to obtain a powder. Methods for drying such mixture are well known to the one skilled in the art and include, without limitation, lyophilisation, freeze-drying, spray-drying, supercritical drying, down-draught evaporation, thin-layer evaporation, centrifugal evaporation, conveyer drying, fluidized bed drying, drum drying or any combination thereof.

In a further particular embodiment, the composition of the invention comprises at least one recombinant cell expressing a polyester hydrolase of the invention, or an extract thereof. An *"extract of a cell"* designates any fraction obtained from a cell, such as cell supernatant, cell debris, cell walls, DNA extract, enzymes or enzyme preparation or any preparation derived from cells by chemical, physical and/or enzymatic treatment, which is essentially free of living cells. Preferred extracts are enzymatically-active extracts. The composition of the invention may comprise one or several recombinant cells of the invention or extract thereof, and optionally one or several additional cells.

In a particular embodiment, the composition consists or comprises a lyophilized culture medium of a recombinant microorganism expressing and excreting a polyester hydrolase of the invention. In a particular embodiment, the powder comprises the polyester hydrolase of the invention and a stabilizing/solubilizing amount of glycerol, sorbitol or dextrin, such as maltodextrine and/or cyclodextrine, starch, glycol such as propanediol, and/or salt.

### Use of the polyester hydrolase of the invention

It is a further object of the invention to provide methods using a polyester hydrolase of the invention for degrading in aerobic or anaerobic conditions and/or recycling polyester containing material, as plastic products made of or containing polyesters. The polyester hydrolases of the invention are particularly useful for degrading a plastic product comprising PET and/or PCL and/or polyester polyurethane.

It is therefore an object of the invention to use a polyester hydrolase of the invention, or a recombinant cell expressing such polyester hydrolase or extract thereof, or composition for the enzymatic degradation of a PET containing material.

It is therefore an object of the invention to use a polyester hydrolase of the invention, or a recombinant cell expressing such polyester hydrolase or extract thereof, or composition for the enzymatic degradation of a PCL containing material.

It is therefore another object of the invention to use a polyester hydrolase of the invention, or a recombinant cell expressing such polyester hydrolase or extract thereof, or composition for the enzymatic degradation of polyester polyurethane containing material.

It is another object of the invention to provide a method for degrading a plastic product containing at least one polyester, wherein the plastic product is contacted with a polyester hydrolase or host cell or composition of the invention, thereby degrading the plastic product. Advantageously, polyester(s) of the polyester containing material is (are) depolymerized up to monomers and/or oligomers.

In an embodiment of the method of degradation, at least one polyester is degraded to yield repolymerizable monomers and/or oligomers, which are advantageously retrieved in order to be reused.

In a particular embodiment, the plastic product comprises at least one polyester selected from polyethylene terephthalate (PET), polytrimethylene terephthalate (PTT), polybutylene terephthalate (PBT), polyethylene isosorbide terephthalate (PEIT), polylactic acid (PLA), polyhydroxyalkanoate (PHA), polybutylene succinate (PBS), polybutylene succinate adipate (PBSA), polybutylene adipate terephthalate (PBAT), polyethylene furanoate (PEF), polycaprolactone (PCL), poly(ethylene adipate) (PEA), polyethylene naphthalate (PEN), polyester polyurethane and blends/mixtures of these materials, preferably polyethylene terephthalate.

In a preferred embodiment, the polyester containing material comprises PET. It is therefore an object of the present invention to provide a method of degradation of a PET-containing material, wherein said PET-containing material is contacted with a polyester hydrolase of the invention. Advantageously, terephthalic acid (TA) and/or ethylene glycol monomers are retrieved. Alternatively, or in addition, oligomers such as methyl-2-hydroxyethyl terephthalate (MHET), bis(2-hydroxyethyl) terephthalate (BHET), 2-hydroxyethyl benzoate (HEB) and dimethyl terephthalate (DMT) may be retrieved. Said monomers and/or oligomers may be reused, for instance for conditioning, repolymerizing polymers, methanisation, etc.

The invention also relates to a method of producing monomers and/or oligomers from a polyester containing material, comprising exposing a polyester containing material to a polyester hydrolase of the invention having a polyester degrading activity targeting at least one polyester of the polyester containing material, or corresponding recombinant cell or extract thereof, or composition, and optionally recovering monomers and/or oligomers.

The method of the invention is particularly useful for producing monomers selected from monoethylene glycol and terephthalic acid, and/or oligomers selected from methyl-2-hydroxyethyl terephthalate (MHET), bis(2-hydroxyethyl) terephthalate (BHET), 2-hydroxyethyl benzoate (HEB) and dimethyl terephthalate (DMT).

It is therefore an object of the present invention to provide a method of producing terephthalic acid (TA) and/or monoethylene glycol monomers, and/or MHET, BHET, HEB and/or DMT oligomers from PET-containing material, comprising exposing said PET-containing material to a polyester hydrolase of the invention having a PET degrading activity, or corresponding recombinant cell or extract thereof, or composition, and optionally recovering at least one monomer selected from TA and monoethylene glycol, and/or at least one oligomer selected from MHET, BHET, HEB and/or DMT.

It is another object of the present invention to provide a method of producing hexanoic acid and/or caproic acid monomers from PCL-containing material, comprising exposing said PCL-containing material to a polyester hydrolase of the invention having a PCL degrading activity, or corresponding recombinant cell or extract thereof, or composition, and optionally recovering hexanoic acid and/or caproic acid monomers.

It is a further object of the present invention to provide a method of producing diethylene glycol (DEG), adipic acid and/or trimethylol propane monomers from polyurethane-containing material, comprising exposing said polyurethane -containing material to a polyester hydrolase of the invention having a polyurethane degrading activity, or corresponding recombinant cell or extract thereof, or composition, and optionally recovering diethylene glycol (DEG), adipic acid and/or trimethylol propane.

The time required for degrading a polyester containing material may vary depending on the polyester containing material itself (i.e., nature and origin of the plastic product, its composition, shape etc.), the type and amount of polyester hydrolase used, as well as various process parameters (i.e., temperature, pH, additional agents, etc.). One skilled in the art may easily adapt the process parameters to the polyester containing material.

Advantageously, the degrading process is implemented at a temperature comprised between 20°C and 90°C, preferably between 40°C and 80°C, more preferably between 50°C and 75°C, more preferably between 60°C and 75°C, even more preferably at 65°C or at 75°C. More generally, the temperature is maintained below an inactivating temperature, which corresponds to the temperature at which the polyester hydrolase is inactivated and/or the recombinant microorganism does no more synthesize the polyester hydrolase. Particularly, the temperature is maintained below the glass transition temperature (Tg) of the polyester in the polyester containing material. More particularly, the process is implemented in a continuous way, at a temperature at which the polyester hydrolase can be used several times and/or recycled.

Advantageously, the degrading process is implemented at a pH comprised between 5 and 11, preferably at a pH between 6 and 9, more preferably at a pH between 6.5 and 9, even more preferably at a pH between 7.5 and 9. Particularly, the degrading process is implemented at pH 8.

In a particular embodiment, the polyester containing material may be pretreated prior to be contacted with the polyester hydrolase, in order to physically change its structure, so as to increase the surface of contact between the polyester and the variant of the invention.

Optionally, monomers and/or oligomers resulting from the depolymerization may be recovered, sequentially or continuously. A single type of monomers and/or oligomers or several different types of monomers and/or oligomers may be recovered, depending on the starting polyester containing material.

The recovered monomers and/or oligomers may be further purified, using all suitable purifying methods and conditioned in a re-polymerizable form. Examples of purifying methods include stripping process, separation by aqueous solution, steam selective condensation, filtration and concentration of the medium after the bioprocess, separation, distillation, vacuum evaporation, extraction, electrodialysis, adsorption, ion exchange, precipitation, crystallization, concentration and acid addition dehydration and precipitation, nanofiltration, acid catalyst treatment, semi continuous mode distillation or continuous mode distillation, solvent extraction, evaporative concentration, evaporative crystallization, liquid/liquid extraction, hydrogenation, azeotropic distillation process, adsorption, column chromatography, simple vacuum distillation and microfiltration, combined or not.

The repolymerizable monomers and/or oligomers may then be reused for instance to synthesize polyesters. Advantageously, polyesters of same nature are repolymerized. However, it is possible to mix the recovered monomers and/or oligomers with other monomers and/or oligomers, in order for instance to synthesize new copolymers. Alternatively, the recovered monomers may be used as chemical intermediates in order to produce new chemical compounds of interest.

It is a further object of the invention to provide a polyester containing material in which a polyester hydrolase of the invention and/or a recombinant microorganism expressing and excreting said polyester hydrolase is/are included. In a particular embodiment, such polyester containing material may be a plastic compound, a masterbatch composition and/or a plastic product. For example, a process for preparing such polyester containing material including a polyester degrading activity is disclosed in WO2013/093335. It is thus an object of the invention to provide a plastic compound, a masterbatch composition and/or a plastic product containing a polyester hydrolase of the invention and/or a recombinant cell and/or a composition or extract thereof and at least one polyester. According to an embodiment, the invention provides a plastic product comprising PET and a polyester hydrolase of the invention having a PET degrading activity. According to another embodiment, the invention provides a plastic product comprising PCL and a polyester hydrolase of the invention having a PCL degrading activity. According to another embodiment, the invention provides a plastic product comprising polyester polyurethane and a polyester hydrolase of the invention having a polyester polyurethane degrading activity.

Classically, a polyester hydrolase of the invention may be used in detergent, food, animal feed and pharmaceutical applications.

More particularly, a polyester hydrolase of the invention may be used as a component of a detergent composition. Detergent compositions include, without limitation, hand or machine laundry detergent compositions, such as laundry additive composition suitable for pre-treatment of stained fabrics and rinse added fabric softener composition, detergent composition for use in general household hard surface cleaning operations, detergent compositions for hand or machine dishwashing operations. In a particular embodiment, a polyester hydrolase of the invention may be used as a detergent additive. The invention thus provides detergent compositions comprising a polyester hydrolase of the invention. Particularly, the polyester hydrolase of the invention may be used as a detergent additive in order to reduce pilling and greying effects during textile cleaning.

The present invention is also directed to methods for using a polyester hydrolase of the invention in animal feed, as well as to feed compositions and feed additives comprising a polyester hydrolase of the invention. The terms "feed" and "feed composition" refer to any compound, preparation, mixture, or composition suitable for, or intended for intake by an animal. In another particular embodiment, the polyester hydrolase of the invention is used to hydrolyze proteins, and to produce hydrolysates comprising peptides. Such hydrolysates may be used as feed composition or feed additives.

The invention also relates to a method of surface hydrolysis or surface functionalization of a polyester containing material, comprising exposing a polyester containing material to a polyester hydrolase of the invention, or corresponding recombinant cell or extract thereof, or composition. The method of the invention is particularly useful for increasing hydrophilicity, or water absorbency, of a polyester material. Such increased hydrophilicity may have particular interest in textiles production, electronics and biomedical applications.

### EXAMPLES

### Example 1 - Cloning, production and purification of the polyester hydrolases

The polyester hydrolases were expressed and purified as described in Wei R et al., 2014, ("Functional Characterization and Structural Modeling of Synthetic Polyester-Degrading Hydrolases from Thermomonospora curvata", AMB Express 4:44) and in Wei R et al., 2016, ("Engineered Bacterial Polyester Hydrolases Efficiently Degrade Polyethylene Terephthalate Due to Relieved Product Inhibition." Biotechnol Bioeng 113:1658-1665).

Briefly, the genes were inserted into the pBAD vector for recombinant expression in *E. coli* One Shot TOP10 and the subsequent purification was done through immobilized metal ion affinity chromatography using Ni-NTA (Qiagen GmbH, Hilden, Germany).

### Example 2 - Evaluation of the activity of the polyester hydrolases on PET films (weight loss)

The hydrolytic activity of the polyester hydrolases of SEQ ID N°1 and SEQ ID N°2 were compared with TfCut2 from *Thermobifida fusca* KW3 (reference and production described in Wei R et al, 2014. "Synthetic polyester-hydrolyzing enzymes from thermophilic actinomycetes." Advances in Applied Microbiology 89:267-305 and Roth C et al, 2014. "Structural and functional studies on a thermostable polyethylene terephthalate degrading hydrolase from Thermobifida fusca." Applied Microbiology and Biotechnology). Such activity was determined by measuring the weight loss of amorphous PET films (Goodfellow GmbH, Bad Nauheim, Germany) submitted to each enzyme.

In each reaction vial, a PET film of approximately 3 cm² (about 50 mg) was added with 16.7 µg/cm² of purified enzyme (polyester hydrolase of SEQ ID N°1, polyester hydrolase of SEQ ID N°2 or TfCut2) in 1 M potassium phosphate buffer (pH 8.0) in a total volume of 1.5 mL and incubated for 24 h at different temperatures from 65 to 75°C as described in Wei R et al., 2016. Weight losses of the PET films were determined gravimetrically using an analytic balance. All measurements were made at least in triplicate and results are detailed in Table 1 below.

**Table 1: Percentage of weight loss of PET films after enzymatic hydrolysis by polyester hydrolases of the invention as well as TfCut2 after 24 h at different reaction temperatures, and improvement factors of the performance of such polyester hydrolases of the invention compared to the performance of TfCut2 in same conditions of enzymatic hydrolysis.**

| | | **65°C** | **70°C** | **75°C** |
|---|---|---|---|---|
| Percentage of weight loss of PET films | TfCut2 (reference) | 3.6 % ± 0.1 | 4.8 % ± 0.0 | 0.8 % ± 0.1 |
| | Polyester hydrolase of SEQ ID N°1 | 74.6 % ± 6.8 | 92.3 % ± 7.6 | 59.3 % ± 4.5 |
| | Polyester hydrolase of SEQ ID N°2 | 16.7 % ± 1.1 | 17.6 % ± 1.2 | 17.3 % ± 2.0 |
| Improvement factor compared to TfCu2 | Polyester hydrolase of SEQ ID N°1 | 21.0 | 19.2 | 69.7 |
| | Polyester hydrolase of SEQ ID N°2 | 4.7 | 3.7 | 20.3 |

The polyester hydrolase of the invention SEQ ID N°1 and the polyester hydrolase of the invention SEQ ID N°2 show a PET degrading activity respectively from 19 to 70 times and from 3.7 to 20 times higher compared to TfCut2. The polyester hydrolases of the invention show an even better degrading activity at 75°C compared to TfCut2.

### Example 3 - Evaluation of the activity of the polyester hydrolases on polyester Polyurethane

Recombinant *E. coli* clones harboring the polyester hydrolase genes (SEQ ID N°3 or SEQ ID N°4) encoding the polyester hydrolases of the invention, respectively SEQ ID N°1 and SEQ ID N°2, were grown on agar plates containing 0.5% polyester polyurethane Impranil® DLN-SD (Bayer MaterialScience AG, Leverkusen, Germany) in the presence of 0.2% L-arabinose at 37°C for 48 h. After 48h, clear lysis halos were visible around the clones expressing the active polyester hydrolases of the invention (SEQ ID N°1 or SEQ ID N°2). This confirms the capacity of the polyester hydrolases of the invention to degrade polyester polyurethane.

### Example 4 - Evaluation of the thermostability of the polyester hydrolases of the invention

The thermal stability of the polyester hydrolases of the invention, SEQ ID N°1 and SEQ ID N°2, was determined and compared with TfCut2 thermal stability by the incubation of reaction vials containing the purified enzyme in 1 M potassium phosphate buffer (pH 8.0) at 65°C, 70°C or 75°C for 24 h. A control sample was stored at 4 °C.

The residual enzyme activity was then determined through the monitoring of the change of turbidity of a PCL nanoparticles suspension submitted to enzymatic hydrolysis at 50 °C as described in Wei R et al., 2014, Functional Characterization and Structural Modeling of Synthetic Polyester-Degrading Hydrolases from Thermomonospora curvata, AMB Express 4:44. Results are shown in Table 2 below.

**Table 2: Residual activity of the polyester hydrolases of SEQ ID N°1 and of SEQ ID N°2 and TfCut2 after incubation at 65 °C, 70 °C and 75 °C for 24 h, compared to activity of the control sample for each enzyme after storage at 4°C. Standard deviation of triplicate is indicated.**

| | Temperature | | | |
|---|---|---|---|---|
| Polypeptide tested | **4°C** | **65°C** | **70°C** | **75°C** |
| Polyester hydrolase of SEQ ID N°1 | 100% | 76.1 % ± 3.3 | 40.1 % ± 3.0 | 33.2 % ± 6.3 |
| Polyester hydrolase of SEQ ID N°2 | 100% | 87.8 % ± 5.3 | 58.2 % ± 1.7 | 54.3 % ± 7.3 |
| TfCut2 | 100% | 43.4 % ±4.6 | 18.0 % ± 0.4 | 21.0 % ± 3.3 |

The results show that the polyester hydrolases of SEQ ID N°1 and SEQ ID N°2 are significantly more stable at temperature above 65°C than TfCut2. The polyester hydrolase of SEQ ID N°1 retains about 76%, 40% and 33% of its initial activity following incubation at 65°C, 70°C and 75°C for 24 h, respectively. The polyester hydrolase of SEQ ID N°2 retains about 88%, 58% and 54% of its initial activity at the respective reaction temperatures. Comparatively, TfCut2 only retained about 43%, 18% and 21% of its initial activity at the respective reaction temperatures.

### Example 5 - Evaluation of the activity of polyester hydrolases on PET powder (monomer production)

The depolymerization of amorphous PET powder was performed with the polyester hydrolase of the invention of SEQ ID N°1. 100 mg of PET powder was weighted and introduced in a dialysis tubing. 1 mL of polyester hydrolase at 0.1 mg/mL in 0.1 M potassium phosphate at pH 8 was added in the dialysis tubing before closing it. The dialysis tubing was then introduced in a glass bottle containing 49 mL of 0.1 M potassium phosphate buffer pH 8.

The depolymerization was started by incubating the sample at 65°C and 150 rpm in a Max Q 4450 incubator (Thermo Fisher Scientific, Inc. Waltham, MA, USA).

Aliquots of 150 µL of buffer were sampled regularly. If necessary, samples were diluted in 0.1 M potassium phosphate buffer pH 8. Then, 150 µL of methanol and 6.5 µL of HCl 6 N were added to 150 µL of sample.

After mixing and filtering on 0.45 µm syringe filter, samples were analyzed by Ultra High Pressure Liquid Chromatography (UHPLC) to monitor the liberation of monomers and oligomers: terephthalic acid (TA), MHET and BHET. Chromatography system used was an Ultimate 3000 UHPLC system (Thermo Fisher Scientific, Inc. Waltham, MA, USA) including a pump module, an autosampler, a column oven thermostated at 25°C, and an UV detector at 240 nm. The column used was a Discovery® HS C18 HPLC Column (150 x 4.6 mm, 5 µm, equipped with precolumn, Supelco, Bellefonte, USA). Eluents were 10 mM H₂SO₄ (eluent A), ultra-pure water (eluent B) and methanol (eluent C). TA, MHET and BHET were separated using a gradient of MeOH in water at 1 mM of H₂SO₄. Injection was 20 µL of sample. TA, MHET and BHET were measured according to standard curves prepared from commercial TA and BHET and in house synthetized MHET in the same conditions than samples.

The percentage of hydrolysis of PET powder was calculated based on the ratio of molar concentration at a given time (TA +MHET + BHET) versus the total amount of TA contained in the initial sample. Results show 78% of depolymerization of the PET powder after 30 hours of reaction.

## Claims

1. An isolated polypeptide comprising an amino acid sequence having at least 75%, 80%, 85%, 90%, 95%, 99% or 100% identity to the full length amino acid sequence set forth in SEQ ID N°1, and having a polyester degrading activity.

2. The isolated polypeptide of claim 1, comprising at least one substitution in the amino acid sequence as set forth in SEQ ID N°1, at a position selected from A2, L210, D233 or S255 or any combinations thereof, wherein the positions are numbered by reference to the amino acid sequence set forth in SEQ ID N°1.

3. The isolated polypeptide of claim 1 or 2, comprising at least one substitution in the amino acid sequence as set forth in SEQ ID N°1, selected from A2E, L210F, D233N or S255A or combinations thereof.

4. The isolated polypeptide of any one of the previous claims, comprising at least the combination of substitutions A2E + L210F + D233N + S255A in the amino acid sequence as set forth in SEQ ID N°1.

5. The isolated polypeptide of any one of the previous claims, which is active in a range of pH from 5-11, preferably in a range of pH from 7-10, more preferably in a range of pH from 7.5-9.

6. The isolated polypeptide of any one of the previous claims, which is active in a range of temperatures from 20°C to 90°C, preferably from 40°C to 80°C, more preferably from 60°C to 80°C, even more preferably at 70°C.

7. The isolated polypeptide of any one of the previous claims, which has a polyethylene terephthalate (PET) degrading activity, a polytrimethylene terephthalate (PTT) degrading activity, a polybutylene terephthalate (PBT) degrading activity, a polyethylene isosorbide terephthalate (PEIT) degrading activity, a polylactic acid (PLA) degrading activity, a polyhydroxy alkanoate (PHA) degrading activity, a polybutylene succinate (PBS) degrading activity, a polybutylene succinate adipate (PBSA) degrading activity, a polybutylene adipate terephthalate (PBAT) degrading activity, a polyethylene furanoate (PEF) degrading activity, a polycaprolactone (PCL) degrading activity, a polyethylene naphthalate (PEN) degrading activity, polyester polyurethane degrading activity and/or a poly(ethylene adipate) (PEA) degrading activity, preferably at least a PET degrading activity.

8. A nucleic acid encoding the polypeptide of any one of claims 1 to 7.

9. An expression cassette or vector comprising the nucleic acid of claim 8.

10. A host cell comprising the nucleic acid of claim 8 or the expression cassette or vector of claim 9.

11. A composition comprising the polypeptide as defined in any of claims 1 to 7, or the host cell according to claim 10, or an extract thereof.

12. A method of producing a polypeptide having a polyester degrading activity according to any one of claims 1-7, comprising:
(a) culturing the host cell according to claim 10 under conditions suitable to express the nucleic acid encoding said polypeptide; and, optionally
(b) recovering said polypeptide from the cell culture.

13. A method of degrading a plastic product containing at least one polyester comprising
(a) contacting the plastic product with a polypeptide according to any one of claims 1 to 7 or the host cell according to claim 10 or the composition according to claim 11; and, optionally
(b) recovering monomers and/or oligomers.

14. The method of claim 13, wherein the plastic product comprises at least one polyester selected from polyethylene terephthalate (PET), polytrimethylene terephthalate (PTT), polybutylene terephthalate (PBT), polyethylene isosorbide terephthalate (PEIT), polylactic acid (PLA), polyhydroxy alkanoate (PHA), polybutylene succinate (PBS), polybutylene succinate adipate (PBSA), polybutylene adipate terephthalate (PBAT), polyethylene furanoate (PEF), Polycaprolactone (PCL), poly(ethylene adipate) (PEA), polyethylene naphthalate (PEN), polyester polyurethane and blends/mixtures of these materials, preferably polyethylene terephthalate.

15. Use of a polypeptide of any one of claims 1 to 7, of a recombinant cell of claim 10, or extract thereof, or of a composition of claim 11, for the degradation of a plastic product containing at least one polyester, preferably at least PET.
